(19) Europäisches Patentamt
European Patent Office
Office européen des brevets

(11) Publication number: 0 022 610 B1

(12) **EUROPEAN PATENT SPECIFICATION**

(45) Date of publication of patent specification: **03.09.86**

(21) Application number: **80301648.4**

(22) Date of filing: **20.05.80**

(51) Int. Cl.[4]: **C 07 C 79/46**, C 07 C 76/02, A 01 N 37/48, C 07 C 65/24, C 07 C 51/215, C 07 C 43/29, C 07 C 41/16, C 07 C 39/235, C 07 C 37/66 // C07C51/363

(54) **Process for preparing 2-nitro-5-(substituted-phenoxy) benzoic acids and salts thereof.**

(30) Priority: **22.06.79 US 51254**
**17.08.79 US 67508**

(43) Date of publication of application:
**21.01.81 Bulletin 81/03**

(45) Publication of the grant of the patent:
**03.09.86 Bulletin 86/36**

(84) Designated Contracting States:
**AT BE CH DE FR GB IT LI NL**

(56) References cited:
**GB-A-1 293 540**
**US-A-3 941 830**
**US-A-3 957 852**
**US-A-4 031 131**

(73) Proprietor: **RHONE-POULENC AGROCHIMIE**
**14-20, rue Pierre Baizet**
**F-69009 Lyon (FR)**

(72) Inventor: **Etherington, Robert William**
**403 S. Main Street**
**Pennington New Jersey (US)**
Inventor: **Theissen, Robert James**
**474 Van Holten Road**
**Bridgewater New Jersey (US)**

(74) Representative: **Brachotte, Charles et al**
**RHONE-POULENC AGROCHIMIE 14-20, Rue Pierre Baizet B.P. 9163**
**F-69263 Lyon Cedex 09 (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 022 610 B1

**Description**

This invention is concerned with the manufacture of 2-nitro-5-(substituted-phenoxy) benzoic acid.

It has been propoposed to prepare 2-nitro-5-(substituted-phenoxy) benzoic acids by the Ullman ether synthesis reaction between an alkali metal salt of a substituted phenol and a halonitrobenzoic acid or between a salt of m-hydroxybenzoic acid and substituted phenylhalide. These methods are disadvantageous, because they involve long periods of time for the coupling reaction, the product is solid, and substituted benzoic acids are relatively expensive. It is the discovery of this invention that by using less expensive m-cresol instead of the benzoic acid reactant, the initial coupling reaction time is shortened and the product from the coupling (Ullman) reaction is liquid and readily separated. Overall, the process is commercially feasible and advantageous from the standpoint of time involved and raw material cost.

This invention provides a process for operating a compound of the formula:

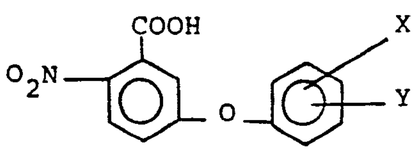

where X and Y are the same or different and are Cl or $CF_3$, that comprises (1) converting m-cresol to an alkali metal salt, (2) reacting said salt with a Cl or $CF_3$ substituted phenyl halide to produce the corresponding 3-(substituted-phenoxy) toluene, (3) oxidizing with air or molecular oxygen the 3-(substituted-phenoxy)-toluene to 3-(substituted-phenoxy)benzoic acid, and (4) nitrating the 3-(substituted-phenoxy)benzoic acid to produce 2-nitro-5-(substituted-phenoxy)benzoic acid.

The m-cresol reactant is readily available commercially. It is converted to its alkali metal salt by a variety of known means for an alkali metal carbonate, bicarbonate, or hydroxide. Preferred alkali metals are Na and K. The salt of m-cresol is reacted with a suitably disubstituted halobenzene, preferably 1,2-dichloro-4-trifluoromethylbenzene and 1,2,4-trichlorobenzene. Such substituted halobenzenes are well known and many are commercially available.

The m-cresol salt formation and the subsequent coupling reaction (Ullman ether synthesis) is carried out in an aprotic organic solvent, such as dimethylformamide, dimethylacetamide, dimethylsulfoxide, sulfolane, hexamethylphosphoric triamide, and formyl piperidine. The coupling reaction can be carried out at temperatures between about 80°C. and about 200°C., preferably between about 140°C. and about 180°C. In general, the coupling reaction is substantially complete in $\frac{1}{4}$ hour. to about 10 hours, depending on the temperature.

A distinct advantage in using m-cresol in the coupling reaction, as compared to the prior art use of the disalt of 3-hydroxybenzoic acid (See U.S. Patent No. 4,031,131), is the shorter reaction time. Additionally, the product is liquid and completely soluble in the aprotic solvent, whereas the product in the disalt method is solid and difficult to isolate. Using the process of this invention, with the coupling product in solution in the aprotic solvent, solid KCl and NaCl byproduct is readily removed by simple filtration and the product is readily isolated by distillation of the filtrate.

In the second step of the process of this invention, the methyl ring substituent (from the m-cresol) is oxidized to carboxyl group. The oxidation can be carried out by any process known in the art of oxidizing a methyl ring substituent with molecular oxygen or air. A particularly feasible process is oxidation of a 20—60 weight percent solution of 3-(substituted-phenoxy) toluene in a lower fatty acid e.g., acetic or in a mixture of lower fatty acid and lower fatty acid anhydride, e.g., acetic acid and acetic anhydride. The air or molecular oxygen oxidation is preferably carried out in the presence of cobalt compounds, such as cobalt acetate and initiators, such as bromine compounds (sodium bromide, cobalt bromide, etc.), acetaldehyde, and methylethyl ketone, generally, oxidation temperatures can be 50—150°C.

After oxidation, product 3-(substituted-phenoxy) benzoic acid is nitrated to produce 2-nitro-5-(substituted-phenoxy) benzoic acid. The nitration reaction is generally carried out at a temperature between about 0°C. and about 70°C. with a nitrating agent. Suitable nitrating agents include nitric acid/sulfuric acid, potassium nitrate/sulfuric acid, and nitric acid/sulfuric acid/acetic anhydride. A cosolvent can advantageously be used, such as ethylene dichloride, methylene dichloride, chloroform, or perchloroethylene.

The crude 2-nitro-5-(substituted-phenoxy) benzoic acid can be purified by recrystallization from the usual aromatic hydrocarbon solvents, such as toluene and xylene or from chlorinated aliphatic hydrocarbons, such as those used as cosolvents in the nitration reaction.

The following examples illustrate the process of this invention and demonstrate a method for product recovery.

Example 1

A mixture of 33 grams KOH (85%, 1/2 mole), 32 g. $H_2O$ and 54.07 g. m-cresol (0.2 mole) were charged to a reactor along with 174.2 g. dimethylacetamide (DMAC). A light brown solution was formed. Vacuum was applied to 50 mm Hg. About 15 minutes later at 60°C. distillate started to come off. After about 8 minutes the temperature had reached 83°C. and 1081.2 g. DMAC and $H_2O$ mixture had been collected. Heat was removed and about 70°C. 1074 g. DMAC was charged. The temperature had dropped to about 50°C. and the reaction mixture solidified indicating that the

mixture was substantially free of water. After raising the temperature to 90°C. the solids melted and vacuum was applied at 80—83°C. Then after about 25 minutea, the contents of the reaction vessel solidified because all the solvent had been stripped. 148 g. of DMAC was added. (173 g. DMAC had been removed) and then an additional 110.7 g. of DMAC was added and vacuum was applied. After about 10 minutes, heating and vacuum were removed leaving dark brown solids. An additional 221.4 g. DMAC was removed and 206.7 g. DMAC was added. The solids went back into solution. Under a nitrogen purge 106.3 g. 1,2-dichloro-4-trifluoromethylbenzene was charged and temperature was raised to 161°C. Seven minutes later, reflux had started with a bloody red suspension resulting. After about 8 minutes, a first one gram aliquot was withdrawn for vapor phase chromatography analysis (VPC). 35 minutes later a second 1.7 g. aliquot was taken, about 24 minutes later a third aliquot was taken, and about 26 minutes later, a fourth aliquot was taken. A bloody red suspension resulted. By using VPC to follow the processs of the reaction it appeared that reaction was virtually complete by the end of 1/2 hour. No high boiling component was made.

The solid (KCl) was filtered off through a fritted funnel. The solids were washed with several portions of DMAC. The combined washes and filtrate was stripped on a rotary evaporator under a vacuum of 7—10 mm Hg at a temperature of 70—90°C. The weight of product after evaporation was 147.5 g. Total conversion was 92% and yield of desired 3-(2'-chloro-4'-trifluoromethylphenoxy) toluene was 84.6%.

For comparison purposes, the following example illustrates a typical coupling reaction using the disalt of hydroxybenzoic acid. The prolonged reaction time will be noted. This is due to difficulty in removing most all water because of the thick nature of the reaction mixture and the fact that the disalt is nearly insoluble.

### Comparative Example 1a

Preparation of 3-(2-chloro-4-(trifluoromethyl) phenoxy benzoic acid. A mixture of meta-hydroxy benzoic acid (966 g., 7.0 moles) and potassium carbonate (966 g., 7.0 moles) in dimethylacetamide (7 l.) was stirred and heated to 155°C. as carbon dioxide was evolved and the dipotassium salt was formed. The temperature was then raised to distill out the water along with solvent. After removing 1500 ml. DMAC, 1500 ml. of fresh DMAC solvent was added back to the pot. On the second distillation, 1000 ml. DMAC were removed and 3000 ml. DMAC added back. Finally, 2000 ml. DMAC were distilled out. The temperature was 168°C. and the reaction consisted of a thick hard to stir slurry of fine solids. The reaction was cooled to 150°C. and 3,4 dichlorobenzotrifluoride (1,505 g., 7.0 moles) was charged through a dropping funnel. The temperature was then raised to 154°C. with vigorous stirring. The reaction was monitored by removing a small sample adding a small amount of 10% sodium hydoxide solution and esterifying with dimethylsulfate. Analysis by VPC then showed the starting disalt material and product esters as well as the 1,4,2-dichloro-4-trifluoromethylbenzene starting material. After 65 hours, the reaction was about 80% complete and after about 90 hours, it was 85% complete. There appeared to be no further reaction on continued heating.

The reaction mixture containing heavy salts of the product was cooled to 100°C., poured onto ice water (3 volumes) and acidified with hydrochloric acid. The solid which formed was filtered, washed with warm water (3 ×) and dried to give 1580 g. (71.3%) of an off white solid mp. 120—123°C. An infrared spectrum confirmed the product.

Several nearly identical reactions were run using dimethylformamide as solvent. The reactions were also very thick and hard to stir. Reaction times varied from about 40 hours to 100 hours depending on the efficiency of water removal during the initial salt formation step. Isolated yields ranged from 50—80%.

### Example 2

Oxidation was carried out using 20 g. 3-(2'-chloro-4'-trfluoromethylphenoxy) toluene (84.4% pure) and a mixture of 1.54 g. $Co(OCCH_3)_2.4H_2O$ and 1.09 g. $CoBr_2.6H_2O$ and the solvent was a mixture of 24 ml. glacial acetic acid and 21.0 g. acetic anhydride. The mixture was heated to 130°C. under 4—5 psig (0,27—0,34 bar $O_2$ pressure. Reaction was complete in 4 hours. Recovered yield of 3-(2'-chloro-4'-trifluoromethylphenoxy) benzoic acid was 83.3% of theory.

### Example 3

The oxidation was carried out using the 84.4% purity starting material used in Example 2 and under the conditions of Example 2, except that 50 ml. glacial acetic acid was used as the solvent. Reaction was again complete in 4 hours, giving a high yield of product.

### Example 4

Preparation of 5-(2'-chloro-4'-trifluoromethyl phenoxy)-2-nitrobenzoic acid. A stirred slurry of 3-(2'-chloro-4'-trifluoromethylphenoxy) benzoic acid (5,679 g., 18 moles) in dichloromethane (11.1.) was cooled to 6°C. and 88% nitric acid (1546 g., 21.6 moles) was added slowly, keeping the temperature below 10°C. Sulfuric acid (3675 g., 36 mole) was then added slowly over 3.5 hours while keeping the temperature below 10°C.

The reaction was monitored by VPC after taking a sample adding a sodium hydroxide solution and esterifying with dimethylsulfate.

The reaction was allowed to warm to about 20—2°C. over 5 hours and checked by VPC. The reaction was largely complete, but stirred overnight to finish off.

Workup involved warming the mixture to about 30°C. and separating the methylene chloride layer. A second portion of warm solvent was used to extract the residue.

The inorganic acid residue still contains some product acid and nitro isomers. This was diluted with water (3 volumes) and the organics dissolved into hot methylene chloride containing about 10% acetone. The organic layer was separated and combined with the other methylene chloride fractions. The whole solution was then warmed to reflux and small additional portions of acetone were added to help maintain solution of the product. The nitro isomers which are fairly insoluble in this solution was filtered, washed and dried to give a gray solid (645 g., 9.9%) m.p. 207—11°C. The filtrate was concentrated and cooled to precipitate out the desired nitro product, and off white yellow solid (4906 g., 75.4%) m.p. 145—154°C. An infrared spectrum confirmed the structure.

The product 2-nitro-5-(2'-chloro-4'-trifluoromethylphenoxy) benzoic acid has a high herbicidal activity. The salts are also herbicidal.

A particular feasible and preferred method of applying the 2-nitro-5-(substituted-phenoxy) benzoic acids is in the salt form. Preferably, the alkali metal salts are used and most preferably the sodium salt. In practice, the alkali metal (sodium) salt is marketed as an aqueous concentrate solution in water containing between about one pound and about 3 pounds active ingredient per gallon.

One way of preparing the aqueous concentrate is to slurry an amount of acid, calculated to give the desired concentration of salt, in water. Then, the slurry of acid is titrated with concentrated aqueous alkali metal hydroxide (NaOH) of about 40—50 weight percent, using adequate agitation, to a pH of 6.5—10, preferably 7—9.

The preferred method for preparing the aqueous concentrate, however, is to add a stoichiometric amount of the acid to an aqueous solution containing an amount of alkali metal hydroxide calculated to give the desired concentration of salt. Then, the pH is adjusted to 6.5—10, preferably 7—9. The final concentration of active ingredient, e.g., sodium 2-nitro-5-(substituted-phenoxy) benzoate, particularly sodium 2-nitro-5-(2'-chloro-4'-trifluoromethyl) benzoate, in the aqueous concentrate solution can be between about 10 weight percent and about 45 weight percent. Since the acid contains some residual nitro isomers which have a relatively low level of herbicidal activity, the purity of the crude acid in terms of the content of desired active ingredient, e.g. 2-nitro-5-(2'-chloro-4'-trifluoromethylphenoxy) benzoic acid, should be considered when calculating the amount of acid to give the desired concentration of salt (Na) in the final concentrates.

The following example demonstrates a typical procedure for converting the acid into an aqueous concentrate of the alkali metal salt. Although the sodium salt is used herein, other alkali metal salts can be made by this general procedure with suitable adjustment of amounts for molecular weight differences.

Example 5

2-Nitro-H-5-(2'-chloro-4'-trifluoromethylphenoxy) benzoic acid (21.43 wt.%, 90% purity) (hereinafter "acid"), 2.38 wt.% NaOH, and 76.19 wt.% water (deionized or containing less than 100 ppm hardness) were used. The formulation is prepared by dissolving sodium hydroxide in the water. Once sodium hydroxide is dissolved, add the acid and stir until dissolved. Then adjust pH with additional NaOH or acid until the pH is 8.0 ± 1.0. The concentration of the sodium salt is 20.74%, by weight active ingredient. Concentration of the sodium salt is equivalent to 224 grams per liter.

The products of the process are useful as agricultural chemicals and more particularly, as herbicides. The compounds may be applied in various ways to achieve the herbicidal action. They can be applied, per se, as solids or in vaporized form, but are preferably applied as the toxic components in pesticidal compositions of the compound and a carrier. The compositions can be applied, as dusts; as liquid sprays, or as gas-propelled sprays and can contain, in additional to a carrier, additives such as emulsifying agents, binding agents, gases compressed to the liquid state, odorants, stabilizers, and the like. A wide variety of liquid and solid carriers can be used. Non-limiting examples of solid carriers include talc, bentonite, diatomaceous earth, pyrophyllite, fullers earth, gypsum, flours derived from cotton seeds and nut shells, and various natural and synthetic clays having a pH not exceeding about 9.5. Non-limiting examples of liquid carriers, include water; organic solvents, such as alcohols, ketones, amides and esters; mineral oils, such as kerosene, light oils, and medium oils and vegetable oils, such as cottonseed oil.

The compounds of this invention are effective herbicides when applied in herbicidal amounts, i.e., at rates between 0.2 and 10 kg. per hectare.

The pesticidal compositions comprising the active ingredient and the carrier may be supplied either as ready-to-use compositions or as concentrates. Concentrates generally contain a higher proportion of the active ingredient than the ready-to-use composition and accordingly, require dilution prior to use. Both the ready-to-use compositions and the concentrates may be in liquid or solid form, i.e. with the active ingredient blended with a liquid or solid carrier, respectively.

The amount of active ingredient in the composition will depend, primarily, upon whether the composition is a concentrate or a ready-to-use composition. Concentrates will generally contain from 5 to 95% by weight, preferably 10 to 80% by weight, of the active ingredient. The concentration of active ingredient in the ready-to-use compositions will vary according to the method of application for the composition in question and to the desired application rate. In general, ready-to-use compositions will contain from 0.001 to 1, preferably 0.01 to 0.1 percent by weight of the

active ingredient. Thus, the compositions may contain from 0.001 to 95% by weight of active ingredient, the actual amount depending upon the nature of the composition and the method by which it shall be applied.

Concentrates may be either liquid or solid and are usually extendable with water to form emulsions or suspensions containing a smaller proportion of the active ingredient. Alternatively, liquid or solid concentrates may be extended with liquid of solid carriers to give the final ready-to-use composition. Liquid concentrates preferably comprise the active ingredient and an emulsifying agent dissolved in a liquid solvent e.g. an organic solvent of the type mentioned above. The emulsifier is suitably an anionic, cationic or non-ionic emulsifier, e.g. sodium dodecylbenzenesulfonate or an ethylene oxide derivative of an alkyl phenol, a mercaptan, an amine or a carboxylic acid. Liquid concentrates may advantageously contain from 10 to 30 weight percent e.g. 25 weight percent of the ingredient e.g. 1 kg. of active ingredient per 4 kg. of concentrate.

Wettable powders are another preferred form of concentrate and these suitably comprise the active ingredient, a finely-divided solid carrier and at least one surfactant to impart wettability or dispersability. Solid carriers which are suitable for preparing wettable powder formulations may be either organic or inorganic in nature. Suitable organic carriers are soybean, walnut, or wood flour or tobacco dust, and suitable inorganic ones are clays of the bentonite, kaolinite, or fuller's earth types; silicas such as diatomaceous earth; silicates such as talc, pyrophyllite, or alkaline earth silicates; and calcium and magnesium carbonates. The carrier may be a single substance or a mixture of finely divided solids. A surfactant or mixture of surfactants is generally present in an amount of 1 to 10 percent by weight of the wettable powder formulation. Suitable dispersing agents are sodium formaldehydenaphthalene sulfonate or sodium lignin sulfonate. Wetting agents include higher alkylaryl sulfonates ("higher alkyl" meaning alkyl of at least 8 carbon atoms) such as calcium dodecylbenzenesulfonate, alcohol sulfates, alkylphenoxyethoxyethoxyethyl sodium sulfonates, sodium dioctyl sulfosuccinate, and ethylene oxide adducts with fatty alcohols, fatty acids, or with higher alkyl-phenols, such as octylphenoxypolyethoxyethanol. Sticking or spreading agents may be included such as glycerol mannitan laurate or a condensate of polyglycerol and oleic acid modified with phthalic anhydride. The active ingredient content of the wettable powder may be in the range of 20 to 80% by weight; however, the preferred range of concentrations is 50% to 80%.

Dusts may be made by incorporating the active ingredient into a solid carrier, such as finely powdered clays, talc, silica, and synthetic silicates, alkaline earth carbonates, and diluents of natural origin such as tobacco dust or walnut shell flour. Granular formulations can be made from similar type solid carriers except that the particle size is larger, in the range of 15 to 60 mesh (U.S. Standard Sieve Series). A small amount of dispersing agent may be incorporated in these solid formulations. The concentration of active ingredients in these dust or granular formulations may be in the range of 1 to 20% by weight. The solid carriers used in these formulations may be essentially inert or they may consist wholly or part of fertilizing materials such as ammonium sulfate, or other ammonium salts, urea, calcium phosphates, potassium chloride or dried blood.

One particularly convenient method for making solid formulations is to treat the solid carrier with the active ingredients dissolved in a solvent and allow the solvent to evaporate off. This results in the carrier which is usually in the form of finely divided particles, being impregnated with the active ingredients. Another method is to apply the mixture of active ingredients in the molten state or by spraying.

The concentration of the active ingredient in the final ready-to-use composition will depend not only upon the application rate desired (generally in the range of 0.2 to 10 kg. per hectare, as mentioned above), but also upon the application method which is to be used. Wettable powders and liquid concentrates are usually applied as aqueous sprays and applied at application rates varying from about 10 to 1500 liters per hectare. With ground equipment, the application rate will generally be in the range of 100 to 500 liters per hectare, whereas aerial spray equipment will generally apply 15 to 80 liters per hectare.

**Claims**

1. A process for preparing a compound of the formula:

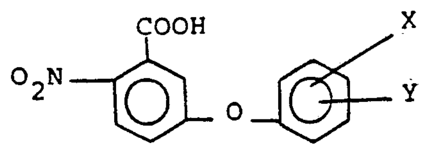

wherein X and Y are the same or different and are Cl or $CF_3$, that comprises

(1) converting m-cresol to an alkali metal salt

(2) reacting said salt with a Cl or $CF_3$ substitued phenyl halide to produce the corresponding 3-(substituted phenoxy) toluene

(3) oxidizing with air or molecular oxygen the 3-(substituted-phenoxy) toluene to 3-(substituted phenoxy) benzoic acid

(4) nitrating the 3-(substituted-phenoxy) benzoic acid to produce 2-nitro-5-(substituted-phenoxy) benzoic acid, and salts thereof.

2. The process of claim 1 wherein X is 2-Cl and Y is 4-$CF_3$ or 4-Cl.

3. The process of claim 2, wherein the oxidation is carried out in the presence of cobalt compound.

4. The process of claim 3 wherein the oxidation

is carried out in the presence of bromine compound.

5. The process of anyone of claim 1 to 4 wherein the oxidation temperature is between 50 and 150°C.

6. The process of any one of claim 1 to 5, wherein the acid of step (4) is (5) converted into an aqueous concentrate of the alkali metal salt.

7. The process of claim 6, wherein said alkali metal is sodium.

8. The process of claim 7, wherin step (5) is carried out by dissolving the acid in an aqueous solution of sodium hydroxide and adjusting the pH to 7—9.

**Patentansprüche**

1. Verfahren zur Herstellung einer Verbindung der formel:

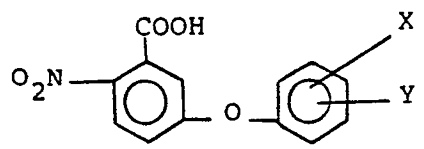

worin X und Y gleich oder unterschiedlich sind und intweder Cl oder $CF_3$ sein können, gekennzeichnet dadurch, daß

(1) m-Kresol in eines seiner. Alkalimetallsalze ungewandelt wird,

(2) dieses Saltz mit einem durch Cl oder $CF_3$ substituierten Phenylhalogenid zur Herstellung eines 3-(substituiertes Phenoxy)toluols umgesetzt wird,

(3) das 3-(substituiertes Phenoxy)toluol zu einer 3-(substituiertes Phenoxy)benzoesäure durch Luft oder Molekularsauerstoff oxydiert wird und

(4) die 3-(substituiertes Phenoxy)benzoesäure nitriert, um zu einer 2-Nitro-5-(substituiertes Phenoxy)bezoesäure (und Salzen von dieser Säure) zu gelangen.

2. Verfahren nach Anspruch 1, worin X Chlor in 2-Stellung und Y $CF_3$ in 4-Stellung oder Cl in 4-Stellung bedeutet.

3. Verfahren nach Anspruch 2, worin oxydation in Anwesenheit von Kobaltverbindung stattfindet.

4. Verfahren nach Anspruch 3, worin oxydation in Anwesenheit von Bromverbindung stattfindet.

5. Verfahren nach einem der Ansprüche 1 bis 5, worin die Oxydationtemperatur zwischen 50 und 150°C liegt.

6. Verfahren nach einem der Ansprüche 1 bis 5, worin die Säure des Verfahrensschrittes (4) als Schritt (5) in ein wäßriges Konzentrat des Alkalimetallsalzes ungewandelt wird.

7. Verfahren nach Anspruch 6, worin das Alkalimetall Natrium ist.

8. Verfahren nach Anspruch 7, worin der Verfahrensschritt (5) durch Auflösen der Säure in einer wäßrigen Lösung von Natriumhydroxid und Einstellen des pH-Wertes auf 7 bis 9 durchgeführt wird.

**Revendications**

1. Procédé de préparation d'un composé de formule:

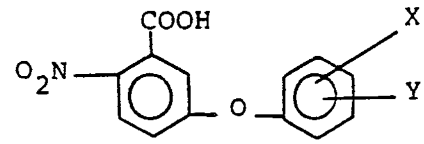

dans laquelle X et Y, identiques ou differentes, représentent Cl ou $CF_3$, qui comprend:

(1) la conversion du métacrésol en un sel de métal alcalin

(2) la réaction du dit sel avec un halogénure de phényle substitué par Cl ou $CF_3$ pour produire le toluène, substitué en 3 par un groupe phénoxy substitué, correspondant

(3) l'oxydation per l'air ou l'oxygène moléculaire du toluène (substitué en position 3 par un groupe phénoxy substitué) en acide benzoïque substitué en position 3 par un groupe phénoxy substitué

(4) la nitration de l'acide benzoïque (substitué en position 3 par un groupe phénoxy substitué) de manière à produire un acide benoïque nitré en position 2 et substitué en position 5 par un groupe phénoxy substitué, et ses sels.

2. Procédé selon la revendication 1, dans lequel X est un chlore en position 2 et Y est un $CF_3$ en position 4 ou un Cl en position 4.

3. Procédé selon la revendication 2, dans lequel l'oxydation est effectuée en présence d'un composé du cobalt.

4. Procédé selon la revendication 3, dans lequel l'oxydation est effectuée en présence d'un composé du brome.

5. Procédé selon l'une des revendications 1 à 4, dans lequel la température d'oxydation est comprise entre 50 et 150°C.

6. Procédé selon l'une des revendications 1 à 5, dans lequel l'acide issu de l'étape (4) est (5) converti en un concentrat aqueux de sel de métal alcalin.

7. Procédé selon la revendication 6, dans lequel ledit métal alcalin est le sodium.

8. Procédé selon la revendication 7, dans lequel l'étape (5) est effectuée par dissolution de l'acide dans une solution aqueuse d'hydroxyde de sodium puis ajustement du pH entre 7 et 9.